# EUROPEAN PATENT SPECIFICATION

(11) **EP 3 011 991 B1**
(45) Date of publication and mention of the grant of the patent: **17.02.2021**
(21) Application number: 15189471.4
(22) Date of filing: 13.10.2015
(51) Int. Cl.: A61M 25/09, A61F 2/966, A61F 2/95

(54) **GUIDE WIRE**
FÜHRUNGSDRAHT
FIL GUIDE

(30) Priority: 22.10.2014 JP 2014214960
(43) Date of publication of application: 27.04.2016
(73) Proprietor: PENTAS Inc., Tokyo, 150-0002 (JP)
(72) Inventor: NISHIGISHI, Makoto c/o ASAHI INTECC CO., LTD., Nagoya-shi, Aichi 463-0024 (JP)
(74) Representative: TBK

(56) References cited:
- EP-A1- 1 400 219
- US-A1- 2006 271 153
- US-A1- 2007 060 996
- US-A1- 2008 255 654
- US-A1- 2008 300 667

## Description

### BACKGROUND OF THE INVENTION

### 1. Field of the Invention

The present disclosure relates to a guide wire for delivering a stent that is received inside a catheter.

Prior art which is related to this field is disclosed in documents US 2006/0271153 A1 showing a system and method for delivering and deploying an occluding device within a vessel, EP 1 400 219 A1 showing an expandable stent and delivery system, US 2008/0255654 A1 showing a system for delivering a stent, US 2008/0300667 A1 showing a system for delivering a stent, and US 2007/0060996 A1 showing a coil shaft.

### 2. Description of the Related Art

A procedure (a stent placement) that recovers blood flow by expanding a stent at a diseased site (an occlusion site of a blood vessel) so that the occlusion site is expanded from the inside is performed. In such a procedure, the stent received inside a catheter is delivered to the diseased site by being pushed forward by a guide wire.

A press member is provided in a guide wire that is used in such a procedure. The stent is advanced by pushing the stent from the back with the press member. Furthermore, a guide wire has been proposed that enables a stent to be drawn back to the side close to the technician by frictional force between an abutting member provided at a portion on a distal end side of a press member (a portion where the stent is mounted on the outside) and the stent (Japanese Unexamined Patent Application Publication (Translation of PCT Application) No. 2013-521022).

### SUMMARY OF THE INVENTION

However, there is a problem in the conventional guide wire described above in that the flexibility of the guide wire is impaired by being provided with the press member and the abutting member. As a result, in some cases, delivering the stent to an end of a meandering blood vessel is disadvantageously difficult.

The present disclosure has been made in response to the problems described above that the conventional art is encountering and an object thereof is to provide a guide wire for delivering a stent with excellent flexibility while being capable of drawing back the stent that is received inside a catheter.

In order to overcome the above problems, a guide wire according to independent claim 1 and subsequent dependent claims 2-5 have been introduce to define solely the invention. An exemplary guide wire, which is capable of delivering a stent received in a catheter, includes a core shaft and an external coil arrangement covering the core shaft. The external coil arrangement includes a first coil portion that abuts against a rear end of the stent inside the catheter and a second coil portion that is positioned on a distal end side of the first coil portion and that abuts against the stent from an inside of the stent. The coil arrangement can include a single integrated coil body or a plurality of coil bodies. I.e., both the first and second coil portions may be comprised in a single integrated coil body, or each of the first and second coil portions may be constituted by one of the coil bodies respectively.

In such a guide wire, since the external coil arrangement is configured by a first coil portion that abuts against the rear end of the stent and a second coil portion that abuts against the stent from the inside of the stent, the stent can be pushed out with the first coil portion and the stent can be drawn back by frictional force between the second coil portion and the inner peripheral surface of the stent.

Note that in the guide wire, the portion (the first coil portion) that pushes the stent and the portion (the second coil portion) that draws back the stent are each configured by a coil. Accordingly, the flexibility of the guide wire can be maintained even when members for performing a pushing-out operation and a drawing-in operation of the stent are provided. As a result, delivering of the stent to the end of the meandering blood vessel can be facilitated.

### BRIEF DESCRIPTION OF THE DRAWINGS

FIG. 1 is an explanatory drawing illustrating a configuration of a guide wire of a first example not forming part of the invention.
FIGS. 2A and 2B are explanatory drawings illustrating states in which the stent is advanced and retracted using the guide wire of the first example. FIG. 2A illustrates a state in which the stent is advanced and FIG. 2B illustrates a state in which the stent is retracted.
FIG. 3 is an explanatory drawing illustrating a configuration of a guide wire of an embodiment of the present disclosure.
FIG. 4 is an explanatory drawing illustrating a configuration of a guide wire of another embodiment of the present disclosure.
FIG. 5 is an explanatory drawing illustrating a configuration of a guide wire **of a useful example for understanding the invention.**
FIG. 6 is an explanatory drawing illustrating a configuration of a guide wire **of a useful example for understanding the invention.** DESCRIPTION OF THE PREFERRED EMBODIMENTS

### A. First Example

Hereinafter, in order to make the details of the present disclosure described above clear, various examples and embodiments of the guide wire of the present disclosure will be described.

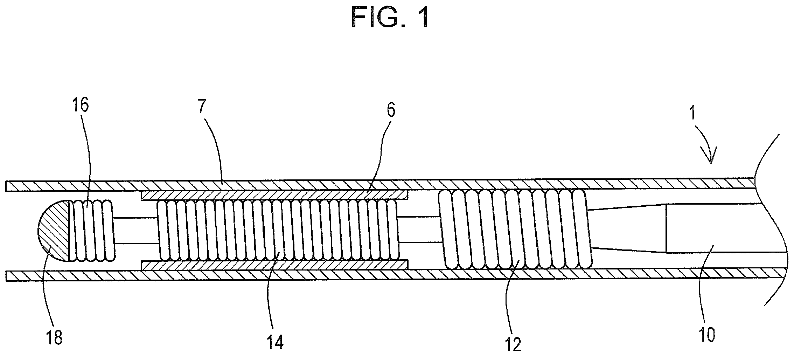

FIG. 1 is an explanatory drawing illustrating a configuration of a guide wire 1 of a first example. The guide wire 1 includes a core shaft 10 and three coil portions (a first coil portion 12, a second coil portion 14, and a third coil portion 16) that are provided on the distal end side of the core shaft 10 (the left side of the drawing). In the guide wire 1, each of the three coil portions is formed as different members. Furthermore, an outside diameter of the second coil portion 14 is set smaller than an outside diameter of the first coil portion 12.

Note that the first coil portion 12 and the second coil portion 14 correspond to an "external coil arrangement".

Among the three coil portions, the first coil portion 12 is disposed on the most proximal end side (the right side of the drawing), the second coil portion 14 is disposed on the distal end side of the first coil portion 12, and the third coil portion 16 is disposed at the most distal end of the core shaft 10. The first coil portion 12 and the second coil portion 14 are each formed of radiolucent metal (stainless steel, for example). On the other hand, the third coil portion 16 is formed of radiopaque metal (platinum, for example) such that the position of the distal end of the guide wire 1 can be perceived in a fluoroscopic image.

The first coil portion 12, the second coil portion 14, and the third coil portion 16 are each joined to the core shaft 10 with a brazing material, an adhesive, or the like (not shown). In FIG. 1, among the joined portions between the three coil portions and the core shaft 10, a distal end brazing portion 18 that joins the distal end of the third coil portion 16 and the distal end of the core shaft 10 to each other is illustrated.

A self-expanding stent 6 is mounted on the outside of the outer peripheral portion of the second coil portion 14. An assembly (a so-called stent delivery system) for delivering the stent 6 to an occlusion site of a blood vessel is configured by mounting the stent 6 on the outside of the second coil portion 14 and by inserting the guide wire 1 and the stent 6 into the bore of the catheter 7. In the above state, the second coil portion 14 abuts against the inner peripheral surface of the stent 6.

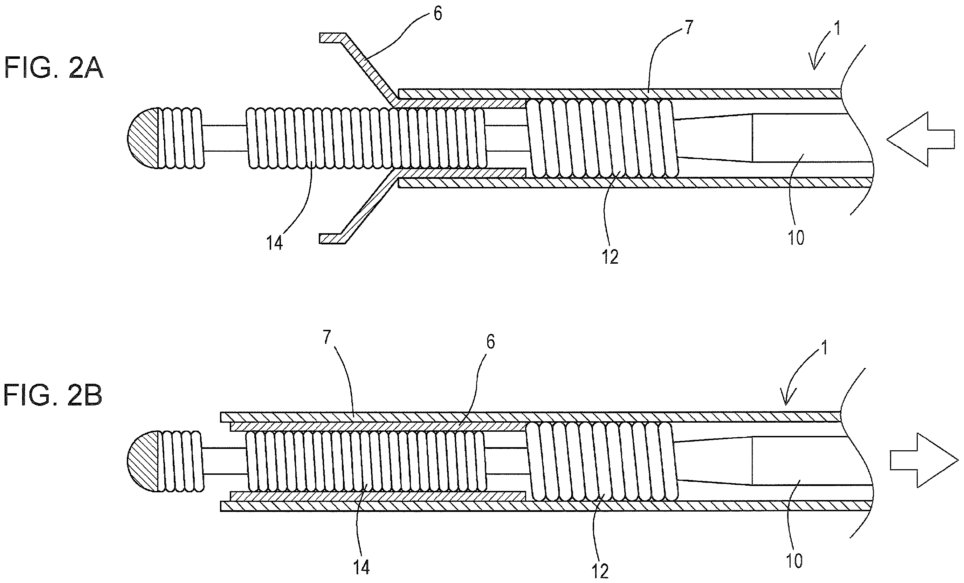

FIGS. 2A and 2B are explanatory drawings illustrating states in which the stent 6 is advanced and retracted using the guide wire 1 FIG. 2A illustrates a state in which the stent 6 is advanced and FIG. 2B illustrates a state in which the stent 6 is retracted.

When the core shaft 10 of the guide wire 1 is pushed towards the distal end side (the left side of the drawing) while the catheter 7 is held, the first coil portion 12 abuts against the rear end of the stent 6 and the stent 6 is pushed by the first coil portion 12 and is moved to the distal end side of the catheter 7. When the stent 6 is pushed out from the distal end of the catheter 7, the stent 6 exposed to the outside of the catheter 7 expands by self-restoring force (see FIG. 2A).

Furthermore, when the guide wire 1 is drawn back towards the proximal end side (the right side of the drawing) while in a state illustrated in FIG. 2A (a state in which a portion of the stent 6 is exposed to the outside of the catheter 7), the stent 6 is moved to the proximal end side of the catheter 7 with frictional force between the stent 6 and the second coil portion 14 and is retrieved into the catheter 7 (see FIG. 2B).

In the above guide wire 1, since the stent 6 not only can be pushed out with the first coil portion 12 but also can be drawn back with the second coil portion 14, fine adjustment of the placing position of the stent 6 can be performed.

Furthermore, in the guide wire 1, the portion (the first coil portion 12) that pushes the stent 6 and the portion (the second coil portion 14) that draws back the stent 6 are each configured by a coil. Accordingly, the flexibility of the guide wire 1 can be maintained even when the members for pushing out and drawing in the stent 6 are provided. As a result, delivering of the stent 6 to the end of the meandering blood vessel can be facilitated.

### B. Exemplary Embodiment

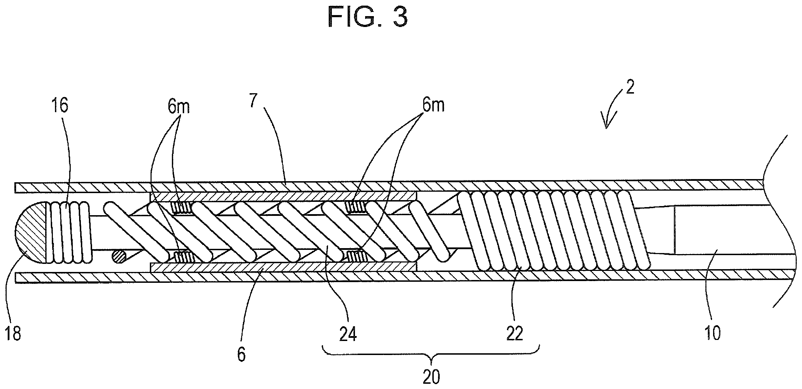

FIG. 3 is an explanatory drawing illustrating a configuration of a guide wire 2 of an embodiment of the present disclosure. The guide wire 2 is different from the guide wire 1 described above in the following points. That is, an external coil arrangement 20 is configured by a first coil portion 22 and a second coil portion 24 that are formed with a plurality of wires in an integrated manner. Furthermore, the first coil portion 22 is formed in a densely wound manner and the second coil portion 24 is formed in a sparsely wound manner.

Note that in the present description, "densely wound" refers to a state in which the adjacent wires of the coil are in contact with each other and "sparsely wound" refers to a state in which the adjacent wires of the coil are not in contact with each other.

Note that the first coil portion 22 and the second coil portion 24 may be formed in an integrated manner using a single wire.

Points other than the above are similar to the guide wire 1 described above. The guide wire 2 of the present embodiment is provided with the third coil portion 16 at the most distal end of the core shaft 10, and the distal end of the core shaft 10 and the distal end of the third coil portion 16 are joined to each other with the distal end brazing portion 18. Furthermore, in a state in which the stent 6 is mounted on the outside of the second coil portion 24 and in which the guide wire 2 and the stent 6 are inserted in the catheter 7, the second coil portion 24 abuts against the inner peripheral surface of the stent 6.

In the above guide wire 2 of the present embodiment, since the first coil portion 22 and the second coil portion 24 are formed in an integrated manner with the plurality of wires, the guide wire 2 that is capable of pushing out and drawing in the stent 6 and that is flexible can be readily fabricated.

Furthermore, since the first coil portion 22 is densely wound, force pushing the stent 6 can be obtained sufficiently. Furthermore, since the second coil portion 24 is sparsely wound, the second coil portion 24 can be readily engaged with the stent 6.

Additionally, since the first coil portion 22 and the second coil portion 24 are formed by the plurality of wires, expansion and contraction of the first coil portion 22 and the second coil portion 24 are suppressed. As a result, the second coil portion 24 can be maintained in a sparsely wound state (a state in which engagement with the stent is facilitated).

Furthermore, since the second coil portion 24 is sparsely wound, an additional effect as below can be obtained. That is, as illustrated in FIG. 3, in a case in which attachments (marker coils 6m) are provided in the stent 6, the marker coils 6m can be engaged between the wires of the second coil portion 24. As a result, the stent 6 can be drawn back easily.

### C. Embodiment

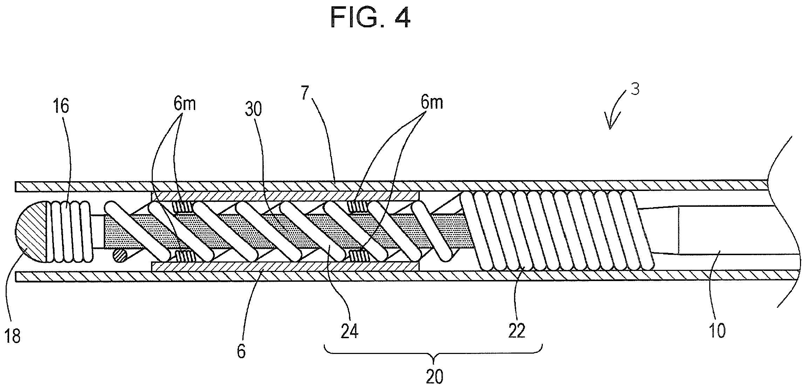

FIG. 4 is an explanatory drawing illustrating a configuration of a guide wire 3 of an embodiment of the present disclosure. The guide wire 3 of the third exemplary embodiment is different from the guide wires of the various exemplary embodiments described above in the following points. That is, a friction member 30 is provided between the external coil arrangement 20, which is formed of the first coil portion 22 and the second coil portion 24, and the core shaft 10. In the present embodiment, a thin rubber sheet is used as the friction member 30.

Note that while in FIG. 4, a case in which the friction member 30 is provided between the external coil arrangement 20 and the core shaft 10 of the guide wire 2 of the embodiment described above is exemplified, a friction member may be provided between the first coil portion 12 and the second coil portion 14, and the core shaft 10 of the guide wire 1 described above (not shown). However, as described above using FIG. 3, from the viewpoint of facilitating fabrication of the guide wire, it is desirable that the first coil portion 22 and the second coil portion 24 are formed in an integral manner as illustrated in FIG. 4.

Points other than the above are similar to the guide wires of the various examples and embodiments described above. In other words, the third coil portion 16 is provided at the most distal end of the core shaft 10, and the distal end of the core shaft 10 and the distal end of the third coil portion 16 are joined to each other with the distal end brazing portion 18. Furthermore, in a state in which the stent 6 is mounted on the outside of the second coil portion 24 and in which the guide wire 3 and the stent 6 are inserted in the catheter 7, the second coil portion 24 abuts against the inner peripheral surface of the stent 6.

In the above guide wire 3 of the present exemplary embodiment, since the friction member 30 is provided between the external coil arrangement 20 and the core shaft 10, the position of the external coil arrangement 20 can be prevented from being displaced with respect to the core shaft 10. Accordingly, force pushing and drawing back the stent 6 can be obtained sufficiently.

Furthermore, as in the present exemplary embodiment, when the second coil portion 24 is formed in a sparsely wound manner, the movement of the wire of the second coil portion 24 on the core shaft 10 is restricted by the friction member 30 and, accordingly, the sparsely wound state can be maintained. As a result, the procedure of engaging the second coil portion 24 with the stent 6 and drawing back the stent 6 can be performed in a reliable manner.

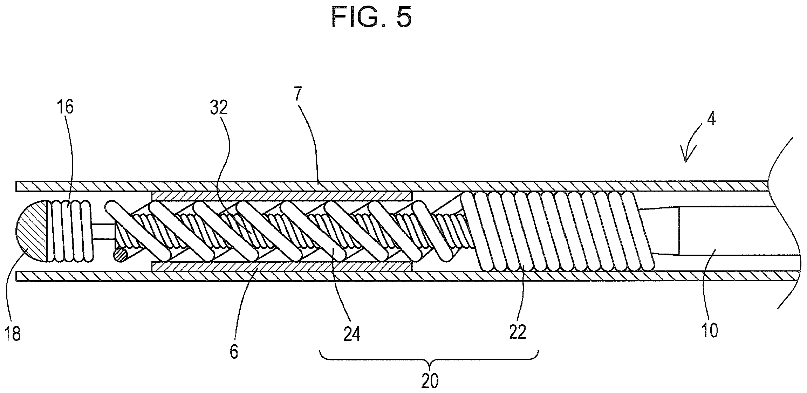

FIG. 5 is an explanatory drawing illustrating a configuration of a guide wire 4 **of a useful example for understanding the invention.** The guide wire 4 **of a useful example for understanding the invention** is different from the guide wires of the various exemplary embodiments described above in the following points. That is, a friction member between the external coil arrangement 20, which is formed of the first coil portion 22 and the second coil portion 24, and the core shaft 10 is configured by an internal coil body 32. The wire diameter of the wire of the internal coil body 32 is smaller than the wire diameter of the wire of the external coil arrangement 20. Furthermore, in FIG. 5, the winding direction of the wire of the internal coil body 32 and the winding direction of the wire of the external coil arrangement 20 are the same.

Note that while in FIG. 5, a case in which the internal coil body 32 is provided between the external coil arrangement 20 and the core shaft 10 of the guide wire 2 described above is exemplified, the internal coil body 32 may be provided between the first coil portion 12 and the second coil portion 14, and the core shaft 10 of the guide wire 1 described above (not shown).

Points other than the above are similar to the guide wires of the various exemplary embodiments described above. That is, the third coil portion 16 is provided at the most distal end of the core shaft 10, and the distal end of the core shaft 10 and the distal end of the third coil portion 16 are joined to each other with the distal end brazing portion 18. Furthermore, in a state in which the stent 6 is mounted on the outside of the second coil portion 24 and in which the guide wire 4 and the stent 6 are inserted in the catheter 7, the second coil portion 24 abuts against the inner peripheral surface of the stent 6.

In the above guide wire 4 of the **useful example for understanding the invention,** since the friction member is a coil body (the internal coil body 32) and the internal coil body 32 is formed of a wire that has a diameter that is smaller than the diameter of the wire of the external coil arrangement 20, in addition to the effects of the various exemplary embodiments described above, the flexibility of the guide wire 4 can be maintained even when the fixing member is provided. As a result, the stent 6 can be reliably delivered to the end of the meandering blood vessel as well.

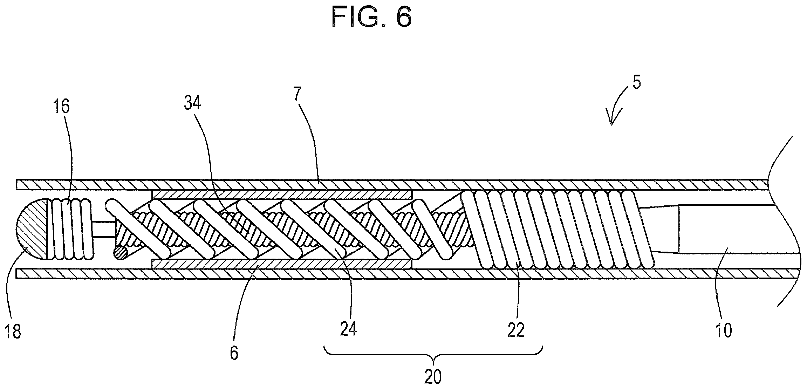

FIG. 6 is an explanatory drawing illustrating a configuration of a guide wire 5 of a **useful example for understanding the invention.** The guide wire 5 of the **useful example for understanding the invention** is different from the guide wires of the various examples and embodiments described above in the following points. That is, an internal coil body 34 is provided between the external coil arrangement 20, which is formed of the first coil portion 22 and the second coil portion 24, and the core shaft 10, and the winding direction of the wire of the internal coil body 34 is opposite to the winding direction of the wire of the external coil arrangement 20.

Points other than the above are similar to the guide wires of the various examples and embodiments described above. In other words, the third coil portion 16 is provided at the most distal end of the core shaft 10, and the distal end of the core shaft 10 and the distal end of the third coil portion 16 are joined to each other with the distal end brazing portion 18. Furthermore, in a state in which the stent 6 is mounted on the outside of the second coil portion 24 and in which the guide wire 4 and the stent 6 are inserted in the catheter 7, the second coil portion 24 abuts against the inner peripheral surface of the stent 6.

In the above guide wire 5 of the **useful example for understanding the invention,** since the winding direction of the wire of the internal coil body 34 is opposite the winding direction of the wire of the external coil arrangement 20, the frictional force between the external coil arrangement 20 and the internal coil body 34 can be increased further. As a result, in addition to the effects of the various examples and embodiments described above, positional displacement of the external coil arrangement 20 with respect to the core shaft 10 can be prevented in a further reliable manner.

While the guide wires of the various examples and embodiments have been described above, the present disclosure is not limited to the exemplary embodiments described above and can be implemented in various manners without departing from the scope of the disclosure. For example, description has been given above that, in the guide wires of the various exemplary embodiments, the outside diameter of the second coil portion is smaller than the diameter of the first coil portion (see FIGS. 1 to 6). However, the outside diameters of the first coil portion and the second coil portion may be by way of an example not forming part of the invention, substantially the same (not shown).

However, the outside diameter of the guide wire having the stent mounted on the outside of the second coil portion can be made smaller when, as in the various exemplary embodiments described above, the outside diameter of the second coil portion is smaller than the outside diameter of the first coil portion. As a result, a catheter with a small outside diameter can be used and the stent can be delivered to a narrow blood vessel as well.

## Claims

1. A guide wire (2, 3) capable of delivering a stent (6) received inside a catheter (7), the guide wire (2, 3) comprising:
a core shaft (10); and
an external coil arrangement (20) covering the core shaft (10), wherein
the external coil arrangement (20) includes
a first coil portion (22) that abuts against a rear end of the stent (6) inside the catheter (7), and
a second coil portion (24) that is positioned on a distal end side of the first coil portion (22) and that abuts against the stent (6) from an inside of the stent (6),
**characterized in that**
the first coil portion (22) and the second coil portion (24) are formed in an integrated manner with a plurality of wires,
the first coil portion (22) is formed in a densely wound manner,
the second coil portion (24) is formed in a sparsely wound manner, and
an attachment (6m) is provided in the stent (6), and can be engaged between the wires of the second coil portion (24).

2. The guide wire (2, 3) according to claim 1, wherein an outside diameter of the second coil portion (24) is smaller than an outside diameter of the first coil portion (22).

3. The guide wire (3) according to claim 1 or 2, wherein a friction member (30) is provided between the external coil arrangement (20) and the core shaft (10).

4. The guide wire (2, 3) according to any one of claims 1 to 3, wherein
the guide wire (2, 3) further comprises a third coil portion (16) disposed on the most proximal end of the core shaft (10),
the first coil portion (22) and the second coil portion (24) are each formed of radiolucent metal, and
the third coil portion (16) is formed of radiopaque metal.

5. The guide wire (2, 3) according to claim 4, wherein
the radiolucent metal is stainless steel, and
the radiopaque metal is platinum.

## Patentansprüche

1. Leitdraht (2, 3), der eine innerhalb eines Katheters (7) eingeführte Gefäßstütze (6) verbringen kann, der Leitdraht (2, 3) umfasst dabei:
eine Kernwelle (10); und
eine externe Windungsanordnung (20), welche die Kernwelle (10) bedeckt, wobei
die externe Windungsanordnung (20) versehen ist mit
einem ersten Windungsabschnitt (22), der gegen ein hinteres Ende der Gefäßstütze (6) innerhalb des Katheters (7) anstößt, und
einem zweiten Windungsabschnitt (24), der an einer distal gelegenen Endseite des ersten Windungsabschnitts (22) angeordnet ist, und von einer Innerseite der Gefäßstütze (6) gegen die Gefäßstütze (6) anstößt,
**dadurch gekennzeichnet, dass**
der erste Windungsabschnitt (22) und der zweite Windungsabschnitt (24) auf integrierte Weise mit einer Vielzahl von Drähten ausgebildet sind,
der erste Windungsabschnitt (22) in einer dichter gewundenen Weise ausgebildet ist,
der zweite Windungsabschnitt (24) in einer spärlicher gewundenen Weise ausgebildet ist, und
eine Halterung (6m) in der Gefäßstütze (6) angeordnet ist, die zwischen den Windungen des zweiten Windungsabschnitts (24) in Eingriff gebracht werden kann.

2. Leitdraht (2, 3) nach Anspruch 1, wobei ein Außendurchmesser des zweiten Windungsabschnitts (24) kleiner als ein Außendurchmesser des ersten Windungsabschnitts (22) ist.

3. Leitdraht (3) nach Anspruch 1 oder 2, wobei ein Reibungselement (30) zwischen der externen Windungsanordnung (20) und der Kernwelle (10) angeordnet ist.

4. Leitdraht (2, 3) nach einem der Ansprüche 1 bis 3, wobei
der Leitdraht (2, 3) ferner einen dritten Windungsabschnitt (16) aufweist, der an dem proximal gelegenen Ende der Kernwelle (10) angeordnet ist,
der erste Windungsabschnitt (22) und der zweite Windungsabschnitt (24) jeweils aus einem strahlendurchlässigen Metall ausgebildet sind, und
der dritte Windungsabschnitt (16) aus einem strahlenundurchlässigen Metall ausgebildet ist.

5. Leitdraht (2, 3) nach Anspruch 4, wobei
das strahlendurchlässige Metall rostfreier Stahl ist, und
das strahlenundurchlässige Metall Platin ist.

## Revendications

1. Fil-guide (2, 3) pouvant poser un stent (6) reçu à l'intérieur d'un cathéter (7), le fil-guide (2, 3) comprenant :
un arbre central (10) ; et
un agencement hélicoïdal externe (20) recouvrant l'arbre central (10), dans lequel :
l'agencement hélicoïdal externe (20) comprend :
une première partie hélicoïdale (22) qui vient en butée contre une extrémité arrière du stent (6) à l'intérieur du cathéter (7), et
une deuxième partie hélicoïdale (24) qui est positionnée sur un côté d'extrémité distale de la première partie hélicoïdale (22) et qui vient en butée contre le stent (6) depuis un intérieur du stent (6),
**caractérisé en ce que** :
la première partie hélicoïdale (22) et la deuxième partie hélicoïdale (24) sont formées d'une manière intégrée par une pluralité de fils,
la première partie hélicoïdale (22) est formée d'une manière enroulée de manière dense,
la deuxième partie hélicoïdale (24) est formée d'une manière enroulée éparse, et
une fixation (6m) est prévue dans le stent (6), et peut être mise en prise entre les fils de la deuxième partie hélicoïdale (24).

2. Fil-guide (3) selon la revendication 1, dans lequel un diamètre externe de la deuxième partie hélicoïdale (24) est inférieur à un diamètre externe de la première partie hélicoïdale (22).

3. Fil-guide (2, 3) selon la revendication 1 ou 2, dans lequel un élément de friction (30) est prévu entre l'agencement hélicoïdal externe (20) et l'arbre central (10).

4. Fil-guide (2, 3) selon l'une quelconque des revendications 1 à 3, dans lequel :
le fil-guide (2, 3) comprend en outre une troisième partie hélicoïdale (16) disposée sur l'extrémité la plus proximale de l'arbre central (10),
la première partie hélicoïdale (22) et la deuxième partie hélicoïdale (24) sont chacune formées avec un métal transparent aux rayons X, et
la troisième partie hélicoïdale (16) est formée avec un métal radio-opaque.

5. Fil-guide (2, 3) selon la revendication 4, dans lequel :
le métal transparent aux rayons X est de l'acier inoxydable, et
le métal radio-opaque est du platine.
